# EUROPEAN PATENT SPECIFICATION

(11) **EP 0 820 777 B1**
(45) Date of publication and mention of the grant of the patent: **18.09.2002**
(21) Application number: 96202096.2
(22) Date of filing: 24.07.1996
(51) Int. Cl.: A61M 5/162

(54) **System for the administration of substances by infusion**
Infusionssystem
Système de perfusion

(43) Date of publication of application: 28.01.1998
(73) Proprietor: HAEMOPHARM INDUSTRY AG, 9490 Vaduz (LI)
(72) Inventor: Valoti, Michele, 6900 Massagno (CH)
(74) Representative: Siniscalco, Fabio

(56) References cited:
- EP-A- 0 567 202
- DE-U- 8 812 460
- DE-U- 9 002 924
- DE-U- 9 201 036
- US-A- 4 826 500

## Description

The present invention relates to a system for the administration by infusion of substances such as, for example, drugs or food supplements, the system being as specified in the preamble of Claim 1.

When administering active substances or solutions of active substances such as, for example, drugs or food supplements, which in the rest of this description will for simplicity's sake be termed active substances, by infusion, it is normal practice to use sterilized bags previously filled with injectable solutions for infusion into a patient. These bags have two openings on their lower edge into which two tubes are inserted; through the first of these tubes, the active substances are transferred from a storage container to the bag where they are mixed with the injectable solution. If the active substances contained in the storage container are, for instance, powders, some of the liquid contained in the bag is squeezed out of the bag into the container where it is used to dissolve the solid substances, after which the resulting solution is passed back into the bag, making use of the increased pressure created in the container.

It is through the second tube that the resulting solution, which in the rest of this description will be described as the final solution, is administered to the patient.

To perform the first stage, that is, transferring the active substances from the storage container into the bag containing the injectable solution, the tube intended for this operation is normally provided, at its free end, with a point having a plurality of holes, which point is positioned centrally inside connector means comprising a female component designed to fit exactly around the neck (the male component) of a container of active substances that are to be transferred into the bag containing the injectable solution. When performing this operation, the female component containing the point is pushed home over the neck of the container; the point simultaneously perforates an elastomeric plug normally used to seal containers of active substances and emerges on the inside of the latter. The system is then simply inverted and the active substance is transferred from the container to the bag, through the abovementioned point and tube.

The German utility model DE-U-8812460 discloses a connector for an infusion system according to the preamble of claim 1.

However, there is a problem with the system described above which cannot be ignored: since the connector component has to fit exactly around the neck of the container, it must be produced in many shapes and sizes, making it necessary to keep a different infusion system for each type of container. The significance of this problem will be understood when it is considered that a great many active substances are administered by infusion and that they are distributed in containers made in an enormous variety of shapes and sizes.

Because these active substances are in daily use by hospital and clinical staff, these staff have to keep permanent supplies of the various transfer devices for the various containers of active substances. Nor should it be forgotten that very often these active substances have to be administered to patients in critical situations in which the person giving emergency aid cannot afford to waste time searching about for a bag with suitable transfer means.

The problem with which the present invention deals is, therefore, how to provide a system for the administration of active substances by infusion that can be used independently of the size and shape of the neck of the container in which the active substance is stored and distributed by pharmaceutical companies, and which therefore solves the above problems.

The problem is solved by a system for the administration of active substances by infusion as specified in the preamble of Claim 1 and having the features claimed in the characterizing part of Claim 1.

The system according to the invention advantageously possesses the features claimed in the dependent claims that follow.

Various preferred embodiments of the present invention will now be described, with reference to the accompanying figures in which:
Figure 1 is a perspective view in partial section of a system for the administration of active substances by infusion.
Figure 2 is a detail of the system depicted in Figure 1, showing how the active substance is transferred from the storage container to the bag for the final solution according to the present invention,
Figure 3 shows a detail of another embodiment illustrating the connector plate and tubular perforator that fit onto the end of the tube through which the active substance is transferred, by screw means, and
Figure 4 shows a detail illustrating an embodiment of the fastening and flow-prevention means according to the invention.

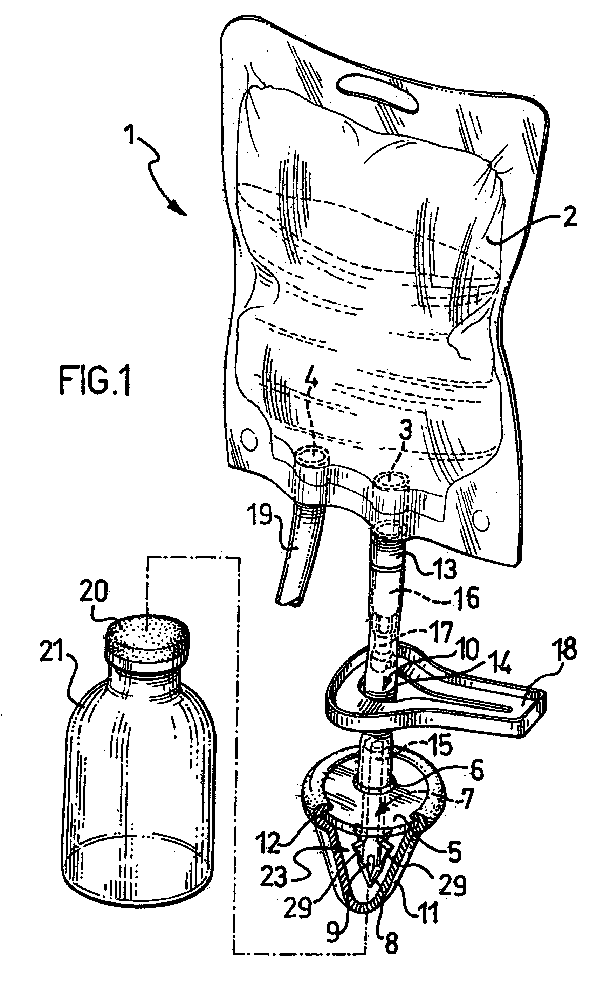

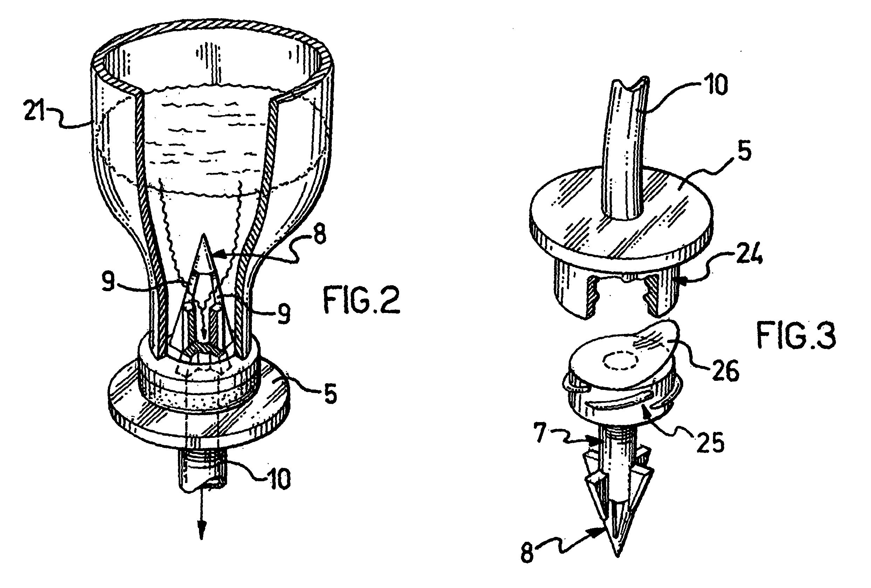

With reference to Figures 1 and 2, the system for administering active substances by infusion, which has the general reference 1, comprises a bag 2 having two openings 3 and 4 formed in its lower edge, a plate 5 of preferably circular shape containing a central hole 6 in which there is force-fitted a tubular perforator 7 having a point 8 at one end provided with a plurality of holes, of which there are two labelled 9 in this example, and barbed fastening means 23, the barbs being formed, in this example, by two pairs of projections 29 on opposite sides. In an alternative form, the plate 5 can be made in one piece with the tubular perforator 7, for example by injection moulding.

The point 8 of the tubular perforator 7 projects from one side of the plate 5, while an end 15 of the tubular perforator 7 projects from the other. This end 15 is a connector component for a tube 10 that allows fluid communication between the tubular perforator 7 and the bag 2. The point 8 of the tubular perforator 7 and the side of the plate 5 from which it projects are protected by a cap 11, made of a soft plastic material and of a generally pointed shape with one end open. The open end of the cap is suitably shaped and contains an annular groove 12 which fits tightly around the edge of the plate 5.

One especially simple embodiment dispenses with the plate 5 and, by way of an abutting member, exploits the fact that the diameter of the end 15 of the tubular perforator 7 is smaller than the external diameter of the tube 10, the former being inserted in the latter. The ring represented by the thickness of the tube 10 that surrounds the end 15 of the tubular perforator 7, thus assumes the function of the abutting member in place of the plate 5.

The tube 10 comprises two portions 13 and 14 connected by a connector component 16 which includes flow prevention means 17. The flow prevention means, which include a breakable line that can be broken to allow fluid communication between the bag 2 and the tubular perforator 7, extend into the interior of the portion 14 of the tube and prevent any passage of fluid through the connector 16, thus isolating the bag 2 from the tubular perforator 7. By this means the sterility of the injectable solution contained in the bag 2 during its storage is guaranteed.

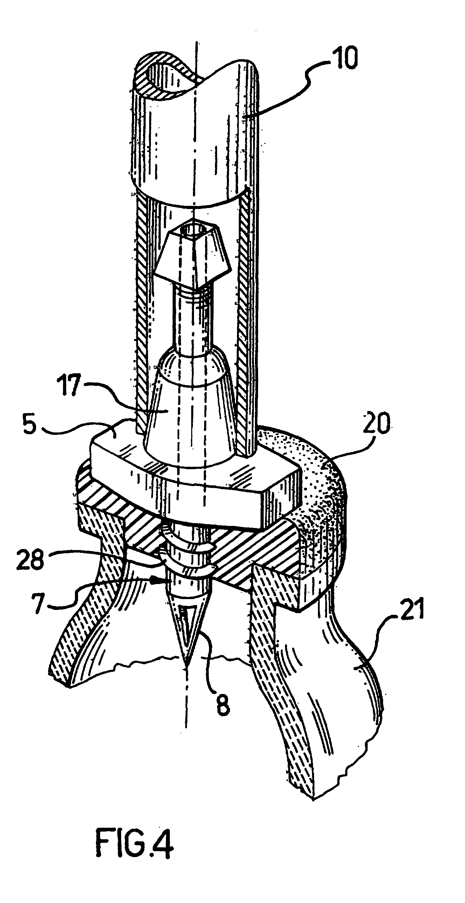

As an alternative, the flow prevention means 17 are made in one piece with the tubular perforator 7, as illustrated in Figure 4, in which case they once again include a breakable line and are essentially elongate. In this embodiment, the connector 16 is no longer necessary and the tube 10 can be formed in one segment.

The portion 14 of the tube 10 also possesses means 18 for pinching the tube: these consist of a clip which can be used to cut off the flow of active substance between the tubular perforator 7 and the bag 2 from the outside at any moment.

The bag 2 is placed in fluid communication with the patient via the opening 4 and a tube 19, to the free end of which a needle (not shown) is fitted for administering the final solution to the patient. Like the tube 10, the tube 19 is also divided into two portions connected by a connector component (not shown) identical in every respect to the connector component 16 described earlier, in order to keep the bag 2 isolated from the external environment for the reasons cited earlier.

When the system according to the invention is to be used, the breakable line of the flow prevention means 17 is broken and, by keeping the system inverted to prevent the escape of the injectable solution from the bag, the fluid connection between the tubular perforator 7 and the bag 2 is thus opened. At this point the cap 11 is detached from the edge of the plate 5 and the tubular perforator 7 is immediately used to perforate a perforable plug 20 of elastomeric material and of predetermined thickness, which seals a container 21 used to store the active substance prior to administration. The tubular perforator 7 of the system is introduced into the fluid until the plate 5 is in abutment against the plug 20. The system is then inverted and, as also illustrated in the example of Figure 2, the active substance begins to flow through the holes 9 into the tubular perforator 7 and then through the tube 10 into the bag 2. The two pairs of projections 29 on opposite sides of the point 8 keep the tubular perforator 7 engaged inside the container 21, so that the container 21 cannot be accidentally disconnected from the bag 2.

On completion of the transfer, the tube 10 is pinched off by the tube pinching means 18, leaving the container 21 suspended from the tube 10. At this point, after briefly homogenising the active substance and the injectable solution in the bag, the final solution can be administered to the patient; to this end the flow prevention means blocking the tube 19 are broken and the solution is injected into the body of the patient.

An exemplary variant of the embodiment described immediately above is illustrated in Figure 3. Mounted on the end of the tube 10, that is the end intended for connection to the plate 5, is a first connector 24, preferably female. This connector is intended for connection to a corresponding second connector 25, which in the preferred case is male: this connector 25 is integral with the tubular perforator 7. The latter, exactly as in the first embodiment, possesses at one end a point 8 that projects from one surface of the plate 5, which point 8 and an opposing end (not shown), serve as a connector component designed to be connected with the corresponding end of the tube 10.

The tubular perforator 7 is fitted with a cap (not shown) and also with a diaphragm 26 to seal off the end 15 of the male component of the screw coupling; the diaphragm 26 is of course removed immediately before the connectors 24 and 25 are joined together.

As an alternative it is obviously possible to form the screw coupling by reversing the positions of the male and female components, so that the end of the tube 10 has a male connector and the tubular perforator 7 has a female connector.

The invention is illustrated in Figure 4. The point 8 of the tubular perforator 7 comprises a self-tapping thread 28 around an axial portion of itself designed to engage with the thickness of the plug 20 of the storage container 21. The tubular perforator 7 is therefore screwed through a half revolution into the plug 20 to secure it to the container 21.

The system according to the present invention has the advantage of being usable with containers of all shapes and sizes and can therefore be bought in large quantities by hospitals or clinics for carrying out infusions of practically all the active substances available on the market.

The presence of the abutment situated at a precise distance from the holes 9 of the tubular perforator 7 also makes it possible to position them in the ideal position inside the container (close to the plug) and hence to transfer a quantity of the active substances from the storage container to the bag.

The embodiment which dispenses with the plate 5 and uses the ring formed by the thickness of the tube 10 as the abutting member, has the particular advantage of being extremely simple and hence allows the manufacturing costs of the entire system to be considerably reduced.

The use of a screw coupling between the tube 10 and the tubular perforator 7 has the additional advantage of supplying the two parts separately so that, should the point or other parts of the system be broken or contaminated as a result, for example, of inattention of the user during the various operations that precede the administration of the final solution to the patient, with this embodiment only one of the two parts need be replaced, rather than the whole system, thus reducing wastage of materials and money.

A person skilled in the art will be able to make many modifications and alterations to the system for the administration of solutions by infusion as described above, in order to satisfy additional particular needs, without however departing from the scope of the invention.

## Claims

1. System for the administration by infusion of liquid or dissolved active substances kept in a necked storage container (21) fitted with a perforable elastomeric material plug (20) of predetermined thickness, comprising a sterilized bag (2), a tubular perforator (7) and a tube (10), said bag (2) containing an injectable solution for infusion into a patient and comprising at one end an opening (3), which tubular perforator (7) extends mostly along its longitudinal axis and is provided at one end with a point (8) for perforating said plug (20) and having holes (9) for the transfer of said active substances from said storage container (21) to said bag (2) to form a final infusion solution, which tube (10) comprises internal flow prevention means (17) that include a breakable line that can be broken to allow fluid communication between said bag (2) and said tubular perforator (7) through said opening (3) and said tube (10), the system being **characterized in that** it comprises a plate (5) projecting from said tubular perforator (7) transversely to the longitudinal axis and intended to abut against said plug (20), said plate (5) being positioned at a predetermined distance from said holes (9) in order that they are correctly positioned during said transfer, in addition to having fastening means (23) including a self-tapping thread (28) formed on an axial portion of said tubular perforator (7) that is intended to engage with the thickness of said plug (20) of said storage container (21).

2. System according to Claim 1, **characterized in that** said plate (5) is integral with said tubular perforator (7).

3. System according to Claim 1 or 2, **characterized in that** said plate (5) is provided with a central hole (6) in which said tubular perforator (7) is force fitted.

4. System according to one of Claims 1 to 3, **characterized in that** said plate (5) is made in one piece with said tubular perforator (7).

5. System according to one of Claims 1 to 4, **characterized in that** said plate (5) is circular.

6. System according to any one of the previous claims, **characterized in that** said flow prevention means (17) are made in one piece with said tubular perforator (7).

7. Method for transferring an active substance in form of powder from a necked storage container (21), fitted with a perforable elastomeric material plug (20) of predetermined thickness, to a sterilized bag (2), filled with an injectable solution for infusion into a patient, said method comprising the steps of:
1) providing a system for administration as depicted in claims 1 to 6;
2) screwing through a half revolution the tubular perforator (7) into the plug (20) for securing it to the container (21);
3) breaking the internal flow prevention means (17);
4) squeezing out of said bag (2) into said necked storage container (21) some of the injectable solution contained in said bag (2); and
5) passing back into said bag (2) the resulting solution of step 4) making use of the increased pressure created in said necked storage container (21).

## Patentansprüche

1. System für die Verabreichung mittels Infusion von flüssigen oder gelösten aktiven Substanzen, die in einem mit einem Hals versehenen Speicherbehälter (21), der mit einem perforierbaren Stopfen (20) aus elastomerem Material einer vorbestimmten Dicke ausgestattet ist, enthalten sind, umfassend einem sterilisierten Beutel (2), einem rohrförmigen Perforierelement (7) und einem Rohr (10), wobei der Beutel (2) eine injizierbare Lösung für die Infusion in einen Patienten enthält und an einem Ende eine öffnung (3) aufweist, wobei sich das röhrförmige Perforierelement (7) zum Großteil entlang seiner Längsachse erstreckt und an einem Ende mit einer Spitze (8) zum Perforieren des Stopfens (20) versehen ist und Löcher (9) für die Übertragung der aktiven Substanzen von dem Speicherbehälter (21) zu dem Beutel (2) aufweist, um eine End-Infusionslösung zu bilden, wobei das Rohr (10) interne Fließverhinderungsmittel (17) umfasst, die eine aufbrechbare Linie aufweisen, welche aufgebrochen werden kann, um eine Fluidverbindung zwischen dem Beutel (2) und dem rohrförmigen Perforierelement (7) durch die Öffnung (3) und das Rohr (10) zu ermöglichen, wobei das System **dadurch gekennzeichnet ist, dass** es eine Platte (5) umfasst, die von dem rohrförmigen Perforierelement (7) quer zu der Längsachse vorspringt und dazu bestimmt ist, an dem Stopfen (20) anzuliegen, wobei die Platte (5) in einem vorbestimmten Abstand zu den Löchern (9) positioniert ist, damit diese während der Übertragung korrekt positioniert sind, und es (das System) außerdem Befestigungsmittel (23) mit einem an einem axialen Abschnitt des rohrförmigen Perforierelements (7) ausgebildeten selbstschneidenden Gewinde (28), das vorgesehen ist, um mit der Dicke des Stopfens (20) des Speicherbehälters (21) in Eingriff zu kommen, aufweist.

2. System gemäß Anspruch 1, **dadurch gekennzeichnet, dass** die Platte (5) integral mit dem rohrförmigen Perforierelement (7) ist.

3. System gemäß Anspruch 1 oder 2, **dadurch gekennzeichnet, dass** die Platte (5) mit einem zentralen Loch (6) versehen ist, in das das rohrförmige Perforierelement (7) mit Kraft eingesetzt ist.

4. System gemäß einem der Ansprüche 1 bis 3, **dadurch gekennzeichnet, dass** die Platte (5) aus einem Stück mit dem rohrförmigen Perforierelement (7) gefertigt ist.

5. System gemäß einem der Ansprüche 1 bis 4, **dadurch gekennzeichnet, dass** die Platte (5) kreisförmig ist.

6. System gemäß einem der vorangehenden Ansprüche, **dadurch gekennzeichnet, dass** die Fließverhinderungsmittel (17) aus einem Stück mit dem rohrförmigen Perforierelement (7) gefertigt sind.

7. Verfahren zum Übertragen einer aktiven Substanzs in Pulverform von einem mit einem Hals versehenen Speicherbehälter (21), der mit einem perforierbaren Stopfen (20) aus elastomerem Material einer bestimmten Dicke ausgestattet ist, zu einem sterilisierten Beutel (2), der mit einer injizierbaren Lösung für die Infusion in einen Patienten gefüllt ist, wobei das Verfahren folgenden Schrite umfasst:
1) Vorsehen eines Verabreichungssystems, wie es in den Ansprüchen 1 bis 6 dargelegt ist,
2) Einschrauben des rohrförmigen Perforierelements (7) in den Stopfen (20) durch eine halbe Drehung, um es an dem Behälter (21) zu befestigen,
3) Aufbrechen der internen Fließverhinderungsmittel (17),
4) Herausdrücken eines Teils der in dem Beutel (2) enthaltenen injizierbaren Lösung aus dem Beutel (2) in den mit einem Hals versehenen Speicherbehälter (21), und
5) Rückübertragen der aus Schritt 4) resultierenden Lösung in den Beutel (2) unter Nutzung des in dem mit einem Hals versehenen Speicherbehälter (21) erzeugten erhöhten Drucks.

## Revendications

1. Un système pour l'administration par perfusion de substances actives liquides ou dissoutes maintenues dans un récipient d'emmagasinage (21) à col pourvu d'un bouchon en matière élastomérique perforable (20) d'une épaisseur prédéterminée, comportant une poche stérilisée (2), un perforateur tubulaire (7) et un tube (10), ladite poche (2) contenant une solution injectable à perfuser dans le corps d'un patient et présentant, à une extrémité, une ouverture (3), lequel perforateur tubulaire (7) s'étend principalement suivant son axe longitudinal et est pourvu à une extrémité d'une pointe (8) destinée à perforer ledit bouchon (2) et ayant des trous (9) pour le transfert desdites substances actives depuis ledit récipient d'emmagasinage (21) jusqu'à ladite poche (2) afin de former une solution finale de perfusion, lequel tube (10) comporte un moyen intérieur (17) de prévention d'écoulement qui présente une ligne de rupture pouvant être rompue pour permettre une communication de fluide entre ladite poche (2) et ledit perforateur tubulaire (7) à travers ladite ouverture (3) et ledit tube (10), le système étant **caractérisé en ce qu'**il comporte une plaque (5) faisant saillie dudit perforateur tubulaire (7) transversalement à l'axe longitudinal et conçue pour porter contre ledit bouchon (20), ladite plaque (5) étant positionnée à une distance prédéterminée desdits trous (9) afin qu'ils soient correctement positionnés pendant ledit transfert, en plus d'avoir des moyens de fixation (23) comprenant un filet auto-taraudeur (28) formé sur une partie axiale dudit perforateur tubulaire (7) qui est destiné à engager l'épaisseur dudit bouchon (20) dudit récipient d'emmagasinage (21).

2. Système selon la revendication 1, **caractérisé en ce que** ladite plaque (5) est intégrée audit perforateur tubulaire (7).

3. Système selon la revendication 1 ou 2, **caractérisé en ce que** ladite plaque (5) est pourvue d'un trou central (6) dans lequel ledit perforateur tubulaire (7) est ajusté à force.

4. Système selon l'une des revendications 1 à 3, **caractérisé en ce que** ladite plaque (5) est réalisée d'une seule pièce avec ledit perforateur tubulaire (7).

5. Système selon l'une des revendications 1 à 4, **caractérisé en ce que** ladite plaque (5) est circulaire.

6. Système selon l'une quelconque des revendications précédentes, **caractérisé en ce que** lesdits moyens (17) de prévention d'écoulement sont réalisés d'une seule pièce avec ledit perforateur tubulaire (7).

7. Procédé pour transférer une substance active sous la forme d'une poudre d'un récipient d'emmagasinage (21) à col, pourvu d'un bouchon (20) en matière élastomérique perforable d'une épaisseur prédéterminée, à une poche stérilisée (2), rempli d'une solution injectable pour une perfusion dans le corps d'un patient, ledit procédé comprenant les étapes dans lesquelles :
1) on utilise un système pour l'administration comme décrit dans les revendications 1 à 6 ;
2) on visse sur un demi-tour le perforateur tubulaire (7) dans le bouchon (20) pour le fixer au récipient (21) ;
3) on rompt les moyens intérieurs (17) de prévention d'écoulement ;
4) on fait passer dans ledit récipient d'emmagasinage (21) à col une partie de la solution injectable contenue dans ladite poche (2) en pressant ladite poche (2) ; et
5) on renvoie dans ladite poche (2) la solution résultante de l'étape 4) en utilisant la pression accrue engendrée dans ledit récipient d'emmagasinage (21) à col.
